Europäisches Patentamt

(19) European Patent Office

Office européen des brevets

(11) Numéro de publication: **0 196 696**
**B1**

(12) **FASCICULE DE BREVET EUROPEEN**

(45) Date de publication du fascicule du brevet:
06.09.89

(51) Int. Cl.⁴: **A 61 B 5/03**, G 01 L 7/08

(21) Numéro de dépôt: 86200335.7

(22) Date de dépôt: 04.03.86

(54) **Capteur implantable de pression intracranienne.**

(30) Priorité: 22.03.85 FR 8504448

(43) Date de publication de la demande:
08.10.86 Bulletin 86/41

(45) Mention de la délivrance du brevet:
06.09.89 Bulletin 89/36

(84) Etats contractants désignés:
AT BE CH DE FR GB IT LI LU NL SE

(56) Documents cités:
DE-A- 1 965 231
FR-A- 2 384 482
US-A- 3 289 134
US-A- 4 023 562
US-A- 4 186 749
US-A- 4 385 636
US-A- 4 393 878

(73) Titulaire: **UNIVERSITE PAUL SABATIER (TOULOUSE III)**
**Etablissement public a caractère scientifique, culturel**
**et professionnel, 118 Route de Narbonne,**
**F-31077 Toulouse Cédex (FR)**

(72) Inventeur: **Lazorthes, Yves, 26 rue d'Auriol,**
**F-31000 Toulouse (FR)**
Inventeur: **Clemente, Gil, Rua Serra**
**Talhada 1102 app. 602 Piedade, 54000 Jaboatao**
**Pernambuco (BR)**
Inventeur: **Aragon, Bernard, 3 allée des Mésanges,**
**Auzeville F-31320 Castanet (FR)**

(74) Mandataire: **Barre, Philippe, Cabinet**
**Barre-Gatti-Laforgue 95 rue des Amidonniers,**
**F-31069 Toulouse Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un capteur de pression destiné à être crânienne pour délivrer un signal électrique représentatif de la pression intracrânienne (pression hydrostatique du liquide céphalo-rachidien intraventriculaire).

On sait que la connaissance de la pression intracrânienne est extrêmement précieuse dans de nombreux cas pour suivre et contrôler l'état clinique d'un patient (traumatismes crâniens, hydrocéphalies, lésions expansives intracrâniennes,...). Il existe à l'heure actuelle essentiellement trois familles de capteurs de mesure de la pression intracrânienne:

a) capteurs nécessitant une manipulation du liquide céphalo-rachidien intraventriculaire ou cisternal,

b) capteurs, dits sous-duraux, destinés à être implantés dans l'espace sous-dural situé entre dure-mère et arachnoide,

c) capteurs extra-duraux, destinés à être implantés sur la dure-mère, entre la boîte crânienne et cette dernière.

Les capteurs de la première famille a) mesurent directement la pression du liquide céphalo-rachidien, qui est retransmise par un cathéter à un élément de transduction. Les capteurs de la deuxième famille b) mesurent la pression intracrânienne, en utilisant comme interface l'arachnoide qui est une membrane très fine et très souple apte à transmettre intégralement les pressions. Dans les deux cas, la pression à mesurer est directement accessible sans distorsion, de sorte que la mesure fournit une information significative sans qu'il soit nécessaire de prendre des précautions particulières, et ce, en utilisant des capteurs de pression de type traditionnel, simplement choisis pour posséder une sensibilité appropriée.

Toutefois, l'implantation des capteurs a) ou b) implique une effraction de la dure-mère, qui exige une intervention chirurgicale beaucoup plus complexe que celle requise par une implantation extradurale c) et qui s'accompagne de risques de complications bien connus des praticiens (lésion parenchymateuse, méningite, fistule du liquide céphalo-rachidien...); en particulier seule la technique d'implantation extradurale est utilisable pour effectuer une surveillance à long terme sans danger.

Les capteurs extraduraux c) mesurent indirectement la pression intracrânienne en utilisant comme interface la dure-mère; compte-tenu des avantages de cette technique (facilité d'implantation, suppression des risques pour le patient, possibilité d'implantation de longue durée...), de nombreux capteurs de ce type ont été conçus et on pourra se reporter aux brevets suivants qui en fournissent des exemples: brevets FR 2 455 735, 2 384 482, 2 274 261, 2 262 953, brevets US 4 246 908, 4 393 878, 4 186 749, brevet DE 1 965 231...

Toutefois, des mesures comparatives ont démontré que la plupart des capteurs de cette famille c) fournissait des informations erronées, sans qu'il existe une corrélation précise entre la valeur mesurée et la pression réelle du liquide céphalo-rachidien (en particulier lorsque l'on passe d'un individu à un autre). Cependant un type de capteurs de cette famille fournit en pratique des mesures significatives: il s'agit de capteurs à compensation tels que décrits en particulier dans les brevets FR 2 455 735, 2 384 482, US 4 393 878, DE 1 965 231. Dans ces capteurs, un diaphragme est appliqué contre la dure-mère pour recevoir, sur une de ses faces, la pression transmise par celle-ci, et un système de compensation soumet la face opposée du diaphragme à une contrepression, laquelle est asservie de façon à ramener constamment le diaphragme dans sa position initiale: le paramètre mesuré est la valeur de la contrepression nécessaire. Un tel système conduit à un capteur extrêmement complexe, qui, même fabriqué avec les technologies les plus récentes, n'est pas implantable dans sa totalité.

En outre, le brevet US 4 186 749 décrit un capteur qui ne présente pas les défauts des capteurs à compensation, tout en fournissant des mesures significatives. Dans ce capteur, le diaphragme sensible à la pression est un diaphragme métallique dont les déformations sont extrêmement réduites. Le défaut essentiel de ce capteur provient de ce qu'il est peu sensible et nécessite un transducteur électronique très sophistiqué qui en augmente considérablement le coût et lui confère un encombrement important en hauteur, empêchant en pratique que ledit capteur puisse être glissé entre la dure-mère et la boîte crânienne. Un tel capteur directement enfoncé et placé dans le trou de trépan conduit à de très graves difficultés pratiques pour assurer un contact stable et satisfaisant avec la dure-mère; en outre il doit nécessairement être ancré sur la boîte crânienne pour demeurer en place.

En conséquence, il n'existe pas actuellement de capteurs implantables en extradural, qui, à la fois fournissent des informations exactes représentatives de la pression intracrânienne et bénéficient d'une structure simple, permettant de les fabriquer à coût réduit et conduisant à un volume compatible avec une implantation totale et facile dans la boîte crânienne, entre cette dernière et la dure-mère.

La présente invention vise à remédier à cette absence et se propose de fournir un capteur de structure simple, facilement implantable en extradural avec une garantie de contact stable et satisfaisant avec la dure-mère, et apte à délivrer, quel que soit l'individu, un signal électrique représentatif de la pression intracrânienne de celui-ci, avec une marge d'erreur ne dépassant pas celle généralement admise par les praticiens (de l'ordre de 5%).

Il est à noter que le capteur visé par l'invention est tout particulièrement intéressant lorsqu'il est implanté en extradural compte tenu des avantages précédemment rappelés de cette technique; toutefois, la structure de ce capteur est compatible avec d'autres modes d'implantation.

Le capteur visé par l'invention comprend:
- un corps d'épreuve possédant un diaphragme déformable porté à sa périphérie par un anneau de garde sensiblement indéformable,
- un détecteur de déformation associé au diaphragme et adapté pour délivrer un signal électrique fonction des déformations dudit diaphragme,
- et des conducteurs connectés au détecteur en vue de transmettre ledit signal électrique.

Selon la présente invention, ledit capteur est caractérisé en ce que:
- le corps d'épreuve composé du diaphragme et de l'anneau de garde est constitué par un ensemble monobloc formé d'un seul tenant en une matière amagnétique de façon à présenter, d'une part, une partie périphérique constituant l'anneau de garde, d'épaisseur appropriée pour être sensiblement indéformable, d'autres part, une partie centrale constituant le diaphragme, d'épaisseur plus faible,
- le corps d'épreuve possède une face frontale continue, dite de transduction, s'étendant au niveau de sa partie centrale constituant le diaphragme ainsi qu'au niveau de sa partie périphérique constituant l'anneau de garde, une cavité située à l'arrière du diaphragme assurant la réduction d'épaisseur au niveau dudit diaphragme,
- les caractéristiques mécaniques d'élasticité de la matière constituant le corps d'épreuve et les caractéristiques dimensionnelles de la partie centrale de celui-ci constituant le diaphragme sont adaptées de sorte que, dans chaque plan de coupe du diaphragme, la flèche maximale de celui-ci, rapportée à la longueur dudit diaphragme dans ce plan de coupe, soit comprise entre 0,01 et 0,03 lorsque ledit diaphragme est soumis à une pression relative uniforme de 30 kilopascals,
- le détecteur de déformation comprend une jauge résistive appliquée à l'intérieur de la cavité précitée sur la face du diaphragme opposée à la face de transduction.

Par «pression relative», on entend de façon habituelle la différence entre les pressions appliquées sur les deux faces opposées du diaphragme.

Par «plan de coupe du diaphragme», on entend un plan orthogonal au plan moyen du diaphragme.

La structure sus-évoquée du capteur a été conçue à l'issue d'études réalisées sur un grand nombre d'échantillons de dure-mères, qui ont permis de mettre en évidence les raisons pour lesquelles il n'y avait pas corrélation entre les mesures et la pression intracrânienne dans la plupart des capteurs extraduraux connus (à l'exception des capteurs à compensation); ces raisons paraissent être essentiellement au nombre de trois.

Une de ces raisons, mise en lumière par ces études est que, au-delà d'un seuil de déformation critique, la dure-mère conditionne une altération de la pression transmise; la relation déformation/pression n'est pas linéaire et dépend de la dure-mère concernée. Dans ces conditions, la pression recueillie par un capteur appliqué sur la dure-mère ne peut pas être reliée de façon significative à la pression intracrânienne si la dure-mère a subi une déformation supérieure au seuil critique sus-évoqué. Les inventeurs ont pu déterminer la valeur de ce seuil critique pour les pressions intracrâniennes maximales susceptibles de s'exercer en pratique (à savoir 30 kilopascals). Ce seuil rapporté à la longeur du diaphragme est de 0,03. De plus, les inventeurs ont pu mettre en évidence qu'il était possible, en demeurant en-dessous de ce seuil, d'avoir un diaphragme de sensibilité suffisante pour que les déformations de celui-ci soient détectées de façon satisfaisante par une simple jauge résistive appliquée sur le diaphragme. A cet effet, le diaphragme est choisi de sorte que, pour la pression maximale de 30 kilopascals, sa flèche rapportée à sa longueur soit supérieure à la valeur limite de 0,01. Ainsi, d'une part, le corps d'épreuve appliqué contre la dure-mère limite les déformations de cette dernière de sorte que les mesures fournissent une représentation exacte de la pression intracrânienne dans tout le champ des pressions rencontrées, et, d'autre part, le transducteur se limite à une simple jauge résistive, très robuste, bénéficiant d'un coût et d'un encombrement réduit. On obtient ainsi un capteur qui associe une grande simplicité structurelle, une facilité d'implantation par simple glissement entre la dure-mère et la boîte crânienne et de bonnes qualités de sensibilité et d'exactitude.

La deuxième raison des erreurs constatées dans la plupart des capteurs connus provient des difficultés pratiques pour assurer un bon contact entre diaphragme et dure-mère, et ce malgré un ancrage par vis dans la boîte crânienne qui est nécessaire dans la plupart de ces capteurs. Au contraire, de par sa structure, le capteur de l'invention peut être fabriqué avec une très faible épaisseur, et sa forme plate permet de le glisser entre la boîte crânienne et la dure-mère, en assurant un contact stable et satisfaisant avec cette dernière, sans vissage ni ancrage.

Le capteur conforme à l'invention peut en particulier être réalisé de sorte que:

- le corps d'épreuve soit en une matière synthétique biocompatible, transparente aux rayons X, ayant un coefficient de Poisson sensiblement compris entre 0,30 et 0,45 et un module d'Young sensiblement compris entre 2,3 et 2,8 gigapascals,
- la partie centrale dudit corps d'épreuve constituant le diaphragme présente une épaisseur sensiblement comprise entre 0,15 et 0,30 mm.

Le corps d'épreuve peut notamment être réalisé, par moulage ou par usinage, en une des matières synthétiques suivantes: polyméthacrylate de méthyle, polycarbonate.

Une autre raison du manque d'exactitude des capteurs extraduraux connus provient de leur constitution et de leur fabrication; dans ces capteurs, le diaphragme est généralement rapporté sur l'anneau de garde qui le maintient (collage ou autre fixation): dans ces conditions il existe inéluctablement une discontinuité entre la surface de transduction du diaphragme et la surface de l'anneau de garde et, lors de l'implantation, des précontraintes apparaissent et induisent des erreurs. Dans le capteur de l'invention, le diaphragme et l'anneau de garde sont d'un seul tenant en un même matériau et la face frontale de transduction (appelée à s'appliquer contre la dure-mère), s'étend de façon continue sur toute la surface du capteur sans discontinuité du rayon de courbure en quelque point que ce soit de cette face.

Selon un mode de réalisation préféré, cette face continue de transduction est plane: la surface de la partie centrale constituant le diaphragme et la surface de la partie périphérique constituant l'anneau de garde sont alors coplanaires.

Par ailleurs, la jauge résistive, qui est associée au diaphragme pour délivrer un signal électrique fonction des déformations de celui-ci, peut en particulier comprendre, de façon connue en soi, quatre résistances électriques, montées en deux demi-ponts et reliées à six conducteurs sortant du corps d'épreuve. On obtient ainsi un capteur très simple sur le plan technologique, qui se prête parfaitement à une implantation intracrânienne; les fils conducteurs peuvent être amenés à passer sous le cuir chevelu pour être reliés à un système d'exploitation de type courant, qui lui-même peut-être implanté ou non.

La description qui suit en référence aux dessins annexés présente à titre d'exemple non limitatif un mode de réalisation de l'invention; sur ces dessins qui font partie intégrante de la présente description:

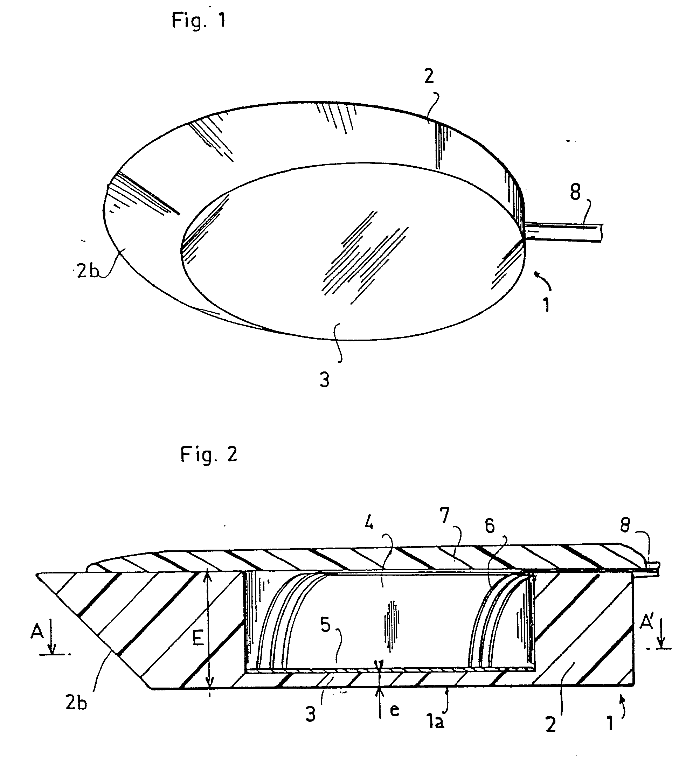

la figure 1 est une vue externe en perspective, à échele dilatée, d'un capteur de pression intracrânienne conforme à l'invention,

la figure 2 en est une coupe par un plan de coupe Pc orthogonal au plan moyen dans lequel s'étend ledit capteur,

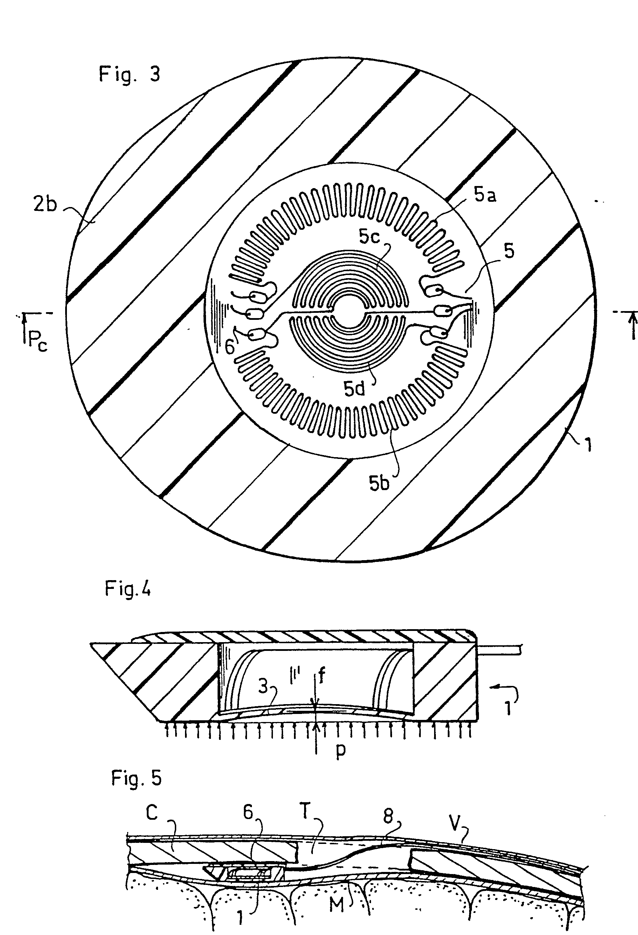

la figure 3 en est une coupe par un plan AA' parallèle à ce plan moyen,

la figure 4 en est un schéma explicatif où la déformation du diaphragme a été très fortement exagérée,

enfin la figure 5 illustre le capteur implanté.

Le capteur de pression représenté à titre d'exemple aux figures est destiné à être implanté contre la dure-mère entre celle-ci et la boîte crânienne en vue de délivrer un signal éléctrique représentatif de la pression hydrostatique du liquide céphalo-rachidien s'exerçant sur la dure-mère.

Ce capteur comprend un corps d'épreuve monobloc 1 qui est fabriqué d'un seul tenant par moulage en polyméthacrylate de méthyle. Ce matériau conditionné lors du moulage par du peroxyde de benzoyle présente de bonnes propriétés de biocompatibilité, d'amagnétisme et de transparence aux rayons X. Son coefficient de Poisson est de 0,4 et son module d'Young de 2,46 gigapascals.

Le corps d'épreuve 1 comprend, d'une part, une partie périphérique 2 d'épaisseur -E- comprise entre 1 mm et 3 mm en particulier de l'ordre de 2 mm, de façon que ses déformations soient négligeables, d'autre part, une partie centrale 3 de forme circulaire dont l'épaisseur -e- est comprise entre 0,15 et 0,30 mm, en particulier épaisseur uniforme de l'ordre de 0,25 mm.

La partie périphérique fait ainsi fonction d'anneau de garde à l'égard de la partie centrale qui joue le rôle de diaphragme déformable.

La face frontale 1a du capteur est appelée à venir s'appliquer contre la dure-mère lors de l'implantation et sera désignée par «face de transduction».

Le corps d'épreuve 1 est réalisé dans un moule à fond plat rectifié, de sorte que cette face 1a soit continue et plane, aussi bien au niveau du diaphragme 3 qu'à celui de l'anneau de garde 2; en d'autres termes, la surface frontale de l'anneau de garde et la surface frontale du diaphragme s'étendent dans un même plan en l'absence de tout discontinuité entre celles-ci.

En l'exemple, sur un côté, l'anneau de garde 2 s'élargit depuis la face de transduction jusqu'à la face opposée de façon à posséder une face latérale biaise 2b adaptée pour faciliter l'insertion du capteur entre la boîte crânienne et la dure-mère lors de l'implantation.

A l'arrière du diaphragme d'épaisseur réduite, le corps d'épreuve 1 possède une cavité 4 de forme cylindrique, d'environ 5 mm de diamètre.

Les caractéristiques d'élasticité du matériau utilisé et l'épaisseur choisie pour le diaphragme limitent les déformations de ce dernier. Ainsi, comme le schématise la figure 4, lorsque le capteur est soumis à une pression relative uniforme de 30 kilopascals, la flèche -f- dans chaque plan de coupe Pc orthogonal au plan du diaphragme est de l'ordre de 0,013 si on la rapporte au rayon dudit diaphragme (constituant sa longueur dans le plan de coupe).

La pression relative intracrânienne étant toujours inférieure à 30 kilopascals (la valeur de 27 kilopascals est généralement admise comme valeur maximale), les dispositions sus-évoquées garantissent que le diaphragme subira, une fois en place, des déformations toujours inférieures au seuil critique en dessous duquel la dure-mère n'introduit pas de distorsions appréciables dans la transmission de la pression du liquide céphalo-rachidien. Il est rappelé que les études des inventeurs ont montré que ce seuil était d'environ 0,030 (flèche maximale rapportée à l'unité de longueur dans chaque plan de coupe Pc).

Par ailleurs, une jauge résistive 5 est collée dans la cavité 4 sur la face du diaphragme opposée à la face de transduction. Les déformations du diaphragme décrit plus haut sont suffisantes pour être détectées de façon parfaitement satisfaisante par une telle jauge.

Cette jauge comprend deux résistances périphériques 5a et 5b qui sont diamétralement opposées et qui serpentent le long de deux arcs de cercle situés à proximité du bord du diaphragme; cette jauge comprend deux autres résistances, centrales, 5c et 5d qui sont disposées en demi-spirales au voisinage du centre du diaphragme à l'opposé l'une de l'autre. Les résistances périphériques 5a, 5b détectent préférentiellement les déformations radiales qui sont maximales en bordure, cependant que les résistances centrales 5c et 5d détectent préférentiellement les déformations tangentielles qui sont maximales au centre.

Ces résistances sont reliées en six points à six fils conducteurs tels que 6, qui permettent de façon traditionnelle de les monter en deux demi-ponts avec, le cas échéant, interposition d'une compensation thermique; il est à noter que la jauge résistive 5 utilisée peut être d'un type connu en soi, dit auto-compensé, qui supprime la nécessité d'une compensation thermique.

La colle utilisée en l'exemple pour fixer la jauge sur le diaphragme est du méthyl-2-cyanoacrylate.

Par ailleurs, la cavité 4 du corps d'épreuve est obturée par un disque-capuchon 7 qui préserve à l'arrière du diaphragme un volume libre autorisant la déformation de celui-ci. Ce disque-capuchon est constitué par une paroi en polyméthacrylate de méthyle, collée en bordure sur l'anneau de garde. Les fils conducteurs 6 sortent du corps d'épreuve, à l'opposé de la face biaise 2b; ces fils passent entre l'anneau de garde et le disque-capuchon 7 et sont protégés à l'extérieur par une gaine 8 jusqu'à une broche de connection (non représentée).

De préférence, le disque-capuchon 7 est étanche et isole la cavité 4 (la déformation du diaphragme conditionnant alors une légère supression dans cette cavité). Il est également possible de prévoir un canal de mise à l'air libre débouchant dans la cavité 4.

La figure 5 montre le capteur conforme à l'invention implanté en extradural. Un trou de trépan T est pratiqué dans la boîte crânienne C de façon à pouvoir insérer le capteur contre la dure-mère M; le capteur est ensuite poussé entre la boîte crânienne et la dure-mère M de façon que sa face de transduction reste appliquée contre cette dernière. La gaine 8 contenant les conducteurs 6 sort par le trou de trépan T et passe sous le cuir chevelu V.

Des essais in vivo ont été effectués sur des chiens dont la pression intracrânienne était amenée à varier au moyen d'un ballonnet gonflable implanté à l'intérieur de la boîte crânienne; ces essais ont montré que les mesures effectuées donnaient une image fidèle de la pression hydrostatique du liquide céphalo-rachidien, avec une erreur relative inférieure à 5%.

Il est à noter que le corps d'épreuve 1 en matériau amagnétique, transparent aux rayons X, évite l'apparition d'artefacts lors d'analyses par Tomodensitométrie ou par I.R.M. («Imagerie par la Résonance Magnétique»).

## Revendications

1. Capteur implantable de pression intracrânienne, comprenant un corps d'épreuve (1) possédant un diaphragme déformable (3) porté à sa périphérie par un anneau de garde sensiblement indéformable (2), un détecteur de déformation (5) associé au diaphragme et adapté pour délivrer un signal électrique fonction des déformations dudit diaphragme, et des conducteurs (6) connectés au détecteur en vue de transmettre ledit signal électrique, ledit capteur étant caractérisé en ce que:
- le corps d'épreuve (1) composé du diaphragme et de l'anneau de garde est constitué par un ensemble monobloc formé d'un seul tenant en une matière amagnétique, de façon à présenter, d'une part, une partie périphérique (2) constituant l'anneau de garde, d'épaisseur appropriée pour être sensiblement indéformable, d'autre part, une partie centrale (3) constituant le diaphragme, d'épaisseur plus faible,
- le corps d'épreuve (1) possède une face frontale continue (1a), dite de transduction, s'étendant au niveau de sa partie centrale (3) constituant le diaphragme ainsi qu'au niveau de sa partie périphérique (2) constituant l'anneau de garde, une cavité (4) située à l'arrière du diaphragme assurant la réduction d'épaisseur au niveau dudit diaphragme,
- les caractéristiques mécaniques d'élasticité de la matière constituant le corps d'épreuve (1) et les caractéristiques dimensionnelles de la partie centrale de celui-ci constituant le diaphragme (3) sont adaptées de sorte que, dans chaque plan de coupe du diaphragme, la flèche maximale de celui-ci, rapportée à la longueur dudit diaphragme dans ce plan de coupe, soit comprise entre 0,01 et 0,03 lorsque ledit diaphragme est soumis à une pression relative uniforme de 30 kilopascals,
- le détecteur de déformation comprend une jauge résistive (5) appliquée à l'intérieur de la cavité (4) précitée sur la face du diaphragme opposée à la face de transduction.

2. Capteur de pression intracrânienne selon la revendication 1, caractérisé en ce que:
- le corps d'épreuve (1) est en une matière synthétique biocompatible, transparente aux rayons X, ayant un coefficient de Poisson sensiblement compris entre 0,30 et 0,45 et un module d'Young sensiblement compris entre 2,3 et 2,8 gigapascals,
- la partie centrale (3) dudit corps d'épreuve constituant le diaphragme présente une épaisseur sensiblement comprise entre 0,15 et 0,30 mm.

3. Capteur de pression intracrânienne selon la revendication 2, caractérisé en ce que la partie périphérique (2) du corps d'épreuve constituant l'anneau de garde présente une épaisseur sensiblement comprise entre 1 et 3 mm.

4. Capteur de pression intracrânienne selon l'une des revendications 1, 2 ou 3, caractérisé en ce que la face continue de transduction (1a) est plane, la surface frontale de la partie centrale (3) constituant le diaphragme et la surface frontale de la partie périphérique (2) constituant l'anneau de garde étant coplanaires.

5. Capteur de pression intracrânienne selon l'une des revendications 1, 2, 3 ou 4, dans lequel la jauge résistive comprend quatre résistances électriques (5a, 5b, 5c, 5d), montées en deux demi-ponts et reliées à six conducteurs (6) sortant du corps d'épreuve (1).

6. Capteur de pression intracrânienne selon la revendication 5, dans lequel la partie centrale (3) formant le diaphragme présente une forme circulaire, caractérisé en ce que:

- deux résistances (5a, 5b) de la jauge sont diamétralement opposées l'une de l'autre et disposées le long de deux arcs de cercle situés à proximité du bord du diaphragme, de façon à détecter préférentiellement les déformations radiales,

- les deux autres résistances (5c, 5d) sont disposées au voisinage du centre du diaphragme à l'opposé l'une de l'autre, de façon à détecter préférentiellement les déformations tangentielles.

7. Capteur de pression selon l'une des revendications 1, 2, 3, 4, 5 ou 6, caractérisé en ce que le corps d'épreuve (1) est réalisé en une des matières synthétiques suivantes: polyméthacrylate de méthyle, polycarbonate.

8. Capteur de pression selon l'une des revendications 1, 2, 3, 4, 5, 6 ou 7, caractérisé en ce que la cavité (4) du corps d'épreuve est obturée par un disque-capuchon (7) agencé pour préserver un volume libre étanche à l'arrière du diaphragme.

9. Capteur de pression selon l'une des revendications précédentes, dans lequel les conducteurs (6) sortant du corps d'épreuve sont protégés par une gaine (8) jusqu'à une broche de connection, caractérisé en ce que la partie périphérique (2) du corps d'épreuve constituant l'anneau de garde possède, du côté opposé à la sortie des conducteurs, une face latérale biaise (2b) adaptée pour faciliter son insertion lors de l'implantation.

**Patentansprüche**

1. Ein implantierbarer intrakranialer Druckaufnehmer mit einem Prüfkörper (1), der eine an seinem Rande durch einen im wesentlichen unverformbaren Schutzring (2) abgestützte verformbare Membran (3) aufweist, einem Verformungsdetektor (5), der mit der Membran in Verbindung steht und so beschaffen ist, dass er ein von den Verformungen der besagten Membran abhängiges elektrisches Signal liefert, und Leitern (6), die mit dem Detektor in Verbindung stehen, um das besagte elektrische Signal zu übertragen, wobei der besagte Aufnehmer dadurch gekennzeichnet ist,

- dass der die Membran und den Schutzring umfassende Prüfkörper (1) in einer integral gefertigten Einheit aus einem nicht magnetischen Material gefertigt wird, wobei er einerseits einen den Schutzring bildenden peripheren Teil (2) aufweist, dessen Dicke so beschaffen ist, dass er im wesentlichen unverformbar ist, und andererseits einen die Membran bildenden Mittelteil (3) geringerer Dicke,

- dass der Prüfkörper (1) eine kontinuierliche Vorderfläche (1a), die sogenannte Transduktionsfläche, aufweist, die sich auf Höhe seines die Membran bildenden Mittelteils (3) sowie auf Höhe seines den Schutzring bildenden peripheren Teils (2) erstreckt, wobei ein hinter der Membran angeordneter Hohlraum (4) Verringerung der Dicke in Höhe der besagten Membran gewährleistet,

- dass die mechanischen Elastizitätsmerkmale des den Prüfkörper (1) bildenden Materials und die Dimensionsmerkmale von dessen die Membran (3) bildenden Mittelteil so beschaffen sind, dass in jeder Schnittebene der Membran deren maximale Durchbiegung im Verhältnis zu der Länge der besagten Membran in dieser Schnittebene zwischen 0,01 und 0,03 beträgt, wenn die besagte Membran einem gleichmässigen relativen Druck von 30 Kilopascal ausgesetzt ist,

- dass der Verformungsdetektor einen an der Innenseite des besagten Hohlraums (4) an der der Transduktionsfläche gegenüberliegenden Membranfläche angeordneten Dehnungsmesser (5) umfasst.

2. Ein intrakranialer Druckaufnehmer nach Anspruch 1, dadurch gekennzeichnet,

- dass der Prüfkörper (1) aus einem biokompatiblen und für Röntgenstrahlen durchlässigen Kunststoff besteht, dessen Poissonscher Koeffizient im wesentlichen zwischen 0,30 und 0,45 und dessen Elastizitätsmodul im wesentlichen zwischen 2,3 und 2,8 Gigapascal beträgt,

- dass der die Membran bildende Mittelteil (3) des besagten Prüfkörpers eine Dicke im wesentlichen zwischen 0,15 und 0,30 mm aufweist.

3. Ein intrakranialer Druckaufnehmer nach Anspruch 2, dadurch gekennzeichnet, dass der den Schutzring bildende periphere Teil (2) des Prüfkörpers eine im wesentlichen zwischen 1 und 3 mm betragende Dicke aufweist.

4. Ein intrakranialer Druckaufnehmer nach einem der Ansprüche 1, 2 oder 3, dadurch gekennzeichnet, dass die kontinuierliche Transduktionsfläche (1a) eben ist, wobei die Vorderfläche des die Membran bildenden Mittelteils (3) und die Vorderfläche des den Schutzring bildenden peripheren Teils (2) koplanar sind.

5. Ein intrakranialer Druckaufnehmer nach einem der Ansprüche 1, 2, 3 oder 4, bei dem der Dehnungsmesser vier elektrische Widerstände (5a, 5b, 5c, 5d) umfasst, die in zwei Halbbrücken angeordnet sind und mit sechs aus dem Prüfkörper (1) austretenden Leitern (6) in Verbindung stehen.

6. Ein intrakranialer Druckaufnehmer nach Anspruch 5, bei dem der die Membran bildende Mittelteil (3) eine Kreisform aufweist, dadurch gekennzeichnet,

- dass zwei Widerstände (5a, 5b) des Dehnungsmessers einander diametral gegenüber und entlang zweier Kreisbogen in der Nähe des Membranrandes angeordnet sind, so dass sie vorzugsweise die radialen Verformungen erfassen,

- dass die beiden anderen Widerstände (5c, 5d) einander gegenüber im Bereich der Membranmitte angeordnet sind, so dass sie vorzugsweise die Tangentialverformungen erfassen.

7. Ein Druckaufnehmer nach einem der Ansprüche 1, 2, 3, 4, 5 oder 6, dadurch gekennzeichnet, dass der Prüfkörper (1) aus einem der folgenden Kunststoffe - Methylpolymethacrylat, Polycarbonat - besteht.

8. Ein Druckaufnehmer nach einem der Ansprüche 1, 2, 3, 4, 5, 6 oder 7, dadurch gekennzeichnet, dass der Hohlraum (4) des Prüfkörpers durch eine Abdeckscheibe (7) abgedeckt ist, die so beschaffen ist, dass hinter der Membran ein dichtes freies Volumen gewahrt bleibt.

9. Ein Druckaufnehmer nach einem der vorstehenden Ansprüche, bei dem die aus dem Prüfkörper austretenden Leiter (6) bis an einen Anschlussstift durch eine Hülse (8) geschützt sind, dadurch gekennzeichnet, dass der den Schutzring bildende periphere Teil (2) des Prüfkörpers an der dem Leiterausgang gegenüberliegenden Seite eine schräge Seitenfläche (2b) aufweist, die so beschaffen ist, dass sie seine Einführung im Zuge der Implantation erleichtert.

## Claims

1. An implantable intracranial pressure sensor comprising a test body (1) with a deformable diaphragm (3) supported at its periphery by a substantially undeformable guard ring (2), with a deformation detector (5) associated with the diaphragm and designed to emit an electrical signal as a function of the deformations of said diaphragm, and with conductors (6) connected to the detector with a view to transmitting said sensor being characterised in that:
- test body (1) comprising the diaphragm and the guard ring consists in an integrally formed unit from a non-magnetic material so as to present, on the one hand, a peripheral part (2) constituting the guard ring of a thickness such as to be substantially undeformable, and on the other hand of a central part (3) constituting the diaphragm and of lesser thickness,
- test body (1) has a continuous frontal face (1a), the so-called transduction face, which extends both to the level of its central part (3) constituting the diaphragm and to the level of its peripheral part (2) constituting the guard ring, with a cavity (4) located at the rear of the diaphragm ensuring the reduction in thickness at the level of said diaphragm,
- the mechanical characteristics of elasticity of the material forming test body (1) and the dimensional characteristics of its central part constituting diaphragm (3) are matched in such a manner that in each sectional plane of the diaphragm, its maximum deflexion relative to the length of said diaphragm in the sectional plane is between 0.01 and 0.03 when said diaphragm is subjected to a uniform relative pressure of 30 kilopascals,
- the deflexion detector comprises a strain gauge (5) applied to the interior of the above cavity (4) on the face of the diaphragm opposite to the transduction face.

2. An intracranial pressure sensor according to claim 1, characterised in that:
- test body (1) is made of a synthetic biocompatible, X-ray transparent material having a Poisson coefficient substantially between 0.30 and 0.45 and a Young's modulus substantially between 2.3 and 2.8 gigapascals,
- central part (3) of said test body constituting the diaphragm has a thickness substantially between 0.15 and 0.30 mm.

3. An intracranial pressure sensor according to claim 2, characterised in that peripheral part (2) of the test body constituting the guard ring has a thickness substantially between 1 and 3 mm.

4. An intracranial pressure sensor according to one of claims 1, 2 or 3, characterised in that continuous transduction face (1a) is plane, the frontal surface of central part (3) constituting the diaphragm and the frontal surface of peripheral part (2) constituting the guard ring being coplanar.

5. An intracranial pressure sensor according to one of claims 1, 2, 3 or 4, wherein the strain gauge comprises four electrical resistors (5a, 5b, 5c, 5d) mounted in two half-bridges and connected to six conductors (6) issuing from test body (1).

6. An intracranial pressure sensor according to claim 5, wherein central part (3) forming the diaphragm exhibits a circular shape, characterised in that:
- two gauge resistors (5a, 5b) are diametrically opposite to one another and arranged along two arcs of a circle situated in the vicinity of the diaphragm edge, so as to preferentially detect the radial deformations,
- the two other resistors (5c, 5d) are arranged in the vicinity of the diaphragm centre opposite one another, so as to preferetially detect the tangential deformations.

7. A pressure sensor according to one of claims 1, 2, 3, 4, 5 or 6, characterised in that test body (1) is made from one of the following synthetic materials: methyl polymethacrylate, polycarbonate.

8. A pressure sensor according to one of claims 1, 2, 3, 4, 5, 6 or 7, characterised in that cavity (4) of the test body is blocked by a cover-disc (7) designed to maintain an impermeable clear volume at the rear of the diaphragm.

9. A pressure sensor according to one of the preceding claims, wherein conductors (6) issuing from the test body are protected by a sheath (8) as far as a connecting pin, characterised in that the peripheral part (2) of the test body constituting the guard ring has, on the side opposite the conductor outlet, an oblique lateral face (2b) designed to facilitate its insertion during implantation.

Fig. 1

Fig. 2

Fig. 3

Fig. 4

Fig. 5